# EUROPEAN PATENT APPLICATION

(11) **EP 2 460 548 A1**
(43) Date of publication of application: **06.06.2012**
(21) Application number: 10193837.1
(22) Date of filing: 06.12.2010
(51) Int. Cl.: A61M 5/158, A61M 5/142

(54) **Medical assembly comprising monitoring device**

(71) Applicant: Roche Diagnostics GmbH, 68305 Mannheim (DE); F. Hoffmann-La Roche AG, 4070 Basel (CH)
(72) Inventor: Müller, Marcel, 4106, Therwil (CH); Moia, Franco, 4402, Frenkendorf (CH); Jeanbourquin, Edgar, 3075, Rüfenacht (CH); Zörgiebel, Florian, 3011, Bern (CH)
(74) Representative: Rentsch Partner AG

(57) **Abstract**

A method of preparing at least parts of a medical device for application and a medical assembly for preparing or storing the at least one disposable unit (1) of the medical device is disclosed. The medical assembly comprises a chemical or physical monitoring device (4) for counting and/or displaying a time period of application and/or remaining before required disposal or replacement of the disposable unit (1). The monitoring device (4) is automatically activated by moving the at least one disposable unit (1) relative to a second unit (2; 9; 18) of the medical assembly for:
- preparing or positioning the disposable unit (1) for use with the medical device;
- assembling the at least one disposable unit (1) and the medical device; and/or
- withdrawing the disposable unit (1) of a storage compartment (3) of the second unit.

## Description

### Field of the Invention

The invention relates to a medical assembly comprising at least one disposable unit, in particular an infusion set assembly comprising a disposable infusion head, and a monitoring device for counting and/or displaying a time period of application and/or remaining before disposal or replacement of the disposable or replaceable unit. Further the invention relates to a method of preparing a medical assembly for use.

### State of the Art

It is a well-known problem in continuous or repetitive use of medical devices that in practice it is difficult to guarantee that disposable materials of the medical device are changed within a time period necessary for reliable operation of the medical device. This is for example a relevant aspect of medical devices for administration of medical or pharmaceutical preparations using disposable injection or infusion units, for measuring health data of a patient using disposable measuring units, for treatment of different patients by the same medical device using disposable or replaceable tool units or even simply for storing disposable or replaceable units of medical devices or parts thereof. An application period and/or expiry date of disposable units is often only provided on the outside of a packaging, whereas the unit itself is not provided with these dates. A specific field of use of medical devices comprising disposable or replaceable units are infusion sets for an insulin pump therapy, in which for example infusion sets in most cases are replaced by diabetics themselves or relatives. Even if these persons are not especially handicapped and despite the fact that they are specifically trained, they often forget to replace disposable units of their medical device, resulting in reduced insulin absorption of the patient's tissue and complications such as for example inflammation or repulsion reactions.

Solutions known in the art include noting relevant data on a separate protocol sheet, providing passive visible indicators also in combination with calendars and the like, e. g. a device similar to a parking disc, or disabling a medical device electronically if for example one unit of the medical device comprises an integral data memory. Also a variety of electronic auxiliary means for medical devices are known, which ether are provided as additional programming or control units, like computers, or may be integrated in a medical device using sensors, processors, LEDs and the like. For example US 7'749'186 B2 discloses a method for determining a service life of a medical administration device, wherein the beginning and elapsing of a service life time is established by sensors for detecting an operating process of the device. A signal is generated which indicates the end of the service life and an output device provides an optical, tactile or acoustical signal announcing the end of service life. The method is focussed on determining the service life of the administration device as a whole, while some units or parts of the device still might be used longer or have to be replaced earlier. In US 2010/022988 A1 a fluid delivery system comprising an infusion set connected to an infusion device is disclosed, which comprises a data exchange device on the infusion device for exchanging data with a data memory on the infusion set. The infusion device may be invalidated via the data exchange device after expiry of a wearing or use period of the infusion set or an expiry of the infusion set may be written on the data memory of the infusion set.

On the other side manifold medical devices are known from the art, which include indication or alarm devices for indicating a failure of the medical device. In particular in the field of medical pump systems alarm devices for indicating leakage, blockage or air enclosure problems are known. In WO 2004/060436 A2 a skin attachment unit of an infusion device is described, which comprises a carrier having a skin-contactin surface and an adhesive layer to attach the infusion device to a skin of a patient. The carrier or the adhesive layer may comprise a material that physically changes, e. g. the color, upon contacting a predetermined fluid, such like insulin. The physical change may indicate leakage of an infusion conduit of the device.

In US 2003/0135159 A1 a drug delivery device for attachment on a user's body is shown, which comprises an internal drug reservoir and an administration system, which administers a liquid drug to a drug delivery outlet via a fluid path. A visual usage indicator comprises a component, which is to be hidden when the device is properly installed on the user's body. The component may include a color that can only be seen by the user if said device is installed improperly. Or the component is realized such, that it is to be removed prior to activating the drug delivery device and the component includes a color that alerts the user to remove the component before the device can be activated.

The principles of indicating or alerting a user of a medical assembly or a medical device comprising disposable or replaceable units known from the art are generally realized ether by complex electronic means or by very simple labeling, which is missing relation to the actual circumstances of application of the medical device. Often the indication devices are not directly linked with the disposable or replaceable unit or part of the medical assembly or the indication devices refer to the durability of materials or medicaments used but not to an optimum application period of a disposable unit, which interacts with its environment during application.

### Object of the invention

It is an object of the present invention to provide a method of preparing a medical assembly, a medical device or at least parts thereof, which facilitates handling and preparation, enables simple application and use of the medical device, makes maintenance easier and increases convenience of a person applying the medical assembly or medical device.

It is a further object of the invention to provide a medical assembly or a medical device comprising at least one disposable unit with reliable indication of an application period or ending of an application period for such a unit, which indicates the status of such unit irrespective of its age, which offers easy inspection of a time period on such unit, which does not require electric or electronic auxiliary means and which offers easy handling.

These and other objects, which will appear from the description below, are achieved by a disposable medical device, a medical assembly and a method of preparing at least parts of a medical device for application as set forth in the appended independent claims. Preferred or advantageous embodiments are defined in the dependent claims.

A disposable medical device or unit for use in combination with a further medical device or second unit comprises a monitoring device for counting and/or displaying a time period of application and/or remaining before required disposal or replacement of the disposable unit. The monitoring device is designed to switch, upon activation, from an inactive state to an active state, where its counts and/or displays the time period of application and/or remaining before required disposal. The disposable medical device or unit is designed such that the monitoring device is automatically activated along with carrying out a preparatory or handling step that is required for preparing the disposable medical device for use.

A method of preparing at least parts of a medical device for application according to the present disclosure refers to medical devices, which comprise at least one disposable unit, like a disposable medical device mentioned above, which has to be prepared for use and/or application in combination with the medical device or for integration into the device. The at least one disposable unit may be part of a medical assembly for preparing or storing the at least one disposable unit. Furthermore the medical device, in particular the at least one disposable device or unit, comprises a chemical or physical monitoring device for counting and/or displaying a time period of application and/or remaining before required disposal or replacement of the disposable unit. That means the monitoring device indicates especially a time period, which starts with the beginning of the preparation of the disposable unit or at the beginning of the application of the disposable unit by the medical device.

In the field of medical devices a disposable unit is to be understood as a unit or a part of the medical device, which shall be discarded or replaced after a certain time of use because of medical, safety, hygienic, or other reasons. A disposal of such units or parts is highly recommended to ensure proper working of the medical device and avoid negative effects on the patients. Other parts or units of the medical device may be reusable, which means they may be used repeatedly with different disposable units. Disposable units are for example infusion sets, injection needles, lancets, cartridges and so on. Depending on the function of the disposable unit, it shall be replaced or disposed after a few hours, a few days or even a few weeks.

According to the disclosed method the monitoring device is automatically activated by moving the at least one disposable device or unit relative to a second unit of the medical assembly for preparing or positioning the disposable device or unit for use with the medical device; for assembling the at least one disposable device or unit and the medical device; and/or for withdrawing the disposable device or unit of a storage compartment of the second unit. Therefore the monitoring device is not activated by a particular, individual activating process with the only intent of starting the monitoring device. Rather the monitoring device is activated by a handling or preparation step, which is mandatory within the regular use and preparation of the medical device for use and which is compulsory accomplished for the designated application of the medical device. Such designated application is for example an infusion of fluids, measuring patient data, or the like. In contrary to such a mandatory and compulsory step the mere step of activating the monitoring device is not required for the intended use of the medical device, which means the device would work properly without activating the monitoring device.

Since the activation of the monitoring device is linked to a compulsory preparation step of the medical device the activation of the monitoring device or associated registration of relevant data for carrying out correct disposal or replacement of the disposable unit may not be forgotten or unintentionally ignored by a patient. Also the method does not only rely on complex electric or electronic systems requiring an energy source. The method disclosed is simple, reliable and convenient for a patient.

A medical assembly, which can realize the method described above, comprises at least two units, which are moveable relative to each other, wherein at least one of the units is a disposable device or unit designed for application in combination with a medical device or for integration into a medical device. Furthermore the medical assembly comprises a chemical or physical monitoring device for counting and/or displaying a time period of application and/or remaining before required disposal or replacement of the disposable or replaceable unit. That means the monitoring device can indicate a remaining time period before the disposable device or unit has the be substituted or a time period, which elapsed since the beginning of preparation of the medical device and the disposable device or unit respectively. In an inactive state of the monitoring device or the disposable device or unit respectively the at least two units take a first position relative to each other and the monitoring device is inactive. In an active state the at least two units take a second position after the units have been moved relative to each other and the monitoring device is active. The monitoring device is automatically activated to count and/or display the time period by movement of the at least two units relative to each other from the first to the second position. The automatic activation is realized by a movement of the at least two units, which is mandatory for correct preparation or application of the medical device as explained above. The activation is realized simultaneously, i. e. automatically, with the obligatory movement of the units of the medical assembly to prepare the disposable device or unit for the medical device.

In one embodiment of a medical assembly the monitoring device is arranged on the disposable or replaceable unit, for example an infusion head unit provided for a medical infusion device as explained in more detail below. The monitoring device can advantageously comprise a pressure sensitive activation mechanism, such that the monitoring device may be activated be generating pressure on at least parts of the monitoring device, e. g. an interaction area on one of the at least two units. This is accomplished for example by connecting a coupler port to an infusion device. The pressure may be generated by frontal abutment or by sliding one upon the other of the at least two units, wherein the monitoring device is at least partly squeezed between the two units.

For example the monitoring device comprises at least one activating chamber including a first reactant and at least one indication chamber including a second reactant. The activating chamber and the indication chamber are separated in the inactive state of the monitoring device and are connected in the active state. In general the activating chamber of the monitoring device is located in the interaction area, such that the first or second unit acts on the interaction area and activates the monitoring device, when moved from first to second position. The interaction area may be provided parallel or perpendicular to the moving direction of the at least two units relative to each other. A connecting conduit can be present between the activating chamber and the indication chamber, which is blocked in inactive state of the monitoring device and which is deblocked in the active state. The connecting conduit may be provided by a simple passage, by a membrane, by a predetermined breaking point, or the like. The blocking can be overcome by generating a pressure in the activation chamber, e. g. by pressing the second unit on the interaction area with the activation chamber. The pressure forces the reactant through the connecting conduit against a blocking force, e. g. a breaking point, membrane resistance, capillary force or the like, or interrupts a separation means arranged between the activating chamber and the indication chamber. The separation means advantageously is arranged within the connecting conduit.

Alternatively the blocking of the connecting conduit may be realized by a separation means arranged on the second unit of the medical assembly, which interacts with the monitoring device or the connecting conduit respectively on the disposable unit and blocks the conduit from outside the monitoring device. Such separation means may be removed from the connecting conduit by the relative movement of the disposable unit relative to the second unit of the medical assembly.

After the connecting conduit has been deblocked the first reactant of the activating chamber flows towards the indication chamber and first and second reactants react with each other such that a visible indication results in the indication chamber. The visible indication can move along an axis of the indication chamber in a predetermined period of time. The movement corresponds to continued reaction of the reactants. Next to the indication chamber a time scale may be provided, which assigns the position of the visible indication within the indication chamber to a period of time since the separation of the chambers has been abolished. Thus the time scale indicates the period of time since the at least two units of the medical assembly have been moved relative to each other to prepare the medical device for application. Also the time scale may indicate the remaining time until the disposable unit has to be removed of the medical device, e. g. by a remaining distance to be overcome by the visual indication within the indication chamber. The visual indication e. g. can be the front line of a color area moving forward within the indication chamber during continuous reaction of first and second reactant.

The time scale may indicate the time that lapsed since activation of the monitoring device and/or the remaining time until the disposable device should be discarded and replaced by a fresh one. The monitoring device and the time scale may be such that that the monitoring ends with the recommended maximum usage time. That is, if the recommended maximum usage time is, for example 3 days, the monitoring device may be designed to operate for three days. Alternatively, the monitoring device may be designed to operate for a longer time period of, for example 4 to 5 days for a recommended maximum usage time of three days. Visual indication, such as symbols or traffic light colors may be provided as part of the time scale to indicate if the recommended maximum usage time is not yet due, is due, or is overdue.

The monitoring device can be sealed into a surface of the disposable unit and advantageously is covered by a tight sealing layer, which may not be interrupted during normal use of the medical device. For example the activating chamber and the indication chamber, and if present also the connecting conduit, are sealed into the surface of the disposable or replaceable unit. That means any reactants of the monitoring device are prevented from interacting with the medical device, the medicament within the device or even the tissue of the user.

In one embodiment the second unit may be realized by a storage unit for storing the first unit, in particular the disposable or replaceable unit. The storing unit comprises a separator means for separating the activating chamber and the indication chamber. In one variation the separator means is provided on a base of the storing unit. The separator means may be tightly attached to or integrated in the base. The monitoring device of the disposable unit rests on the separator means in the first state of the monitoring device. As soon as the disposable unit is taken off the storing unit the monitoring device is released from the separator means and the monitoring device starts indicating the time period as mentioned above. In another variation the separator means is provided on a closing foil of the storing unit and engages with the monitoring device in a closed state of the storing unit. The closing foil may be sealed on an opening of the storing unit. Also the separator means may be sealed to the closing foil. When the closing foil is stripped off the storing unit, the separation means remains on the closing foil. The disposable unit with the monitoring device can stay in the storing unit or may be lifted up together with the separation means and can be detached of the closing foil. Thus the separation means is released from the monitoring device and the indication of the time period may start.

The method as disclosed can advantageously be applied to a medical device, which is realized by an infusion device comprising a disposable unit designed as an infusion head to be placed on a tissue of a patient for administration of medical or pharmaceutical liquid into the tissue. The infusion head may comprise a connected or connectable cannula for insertion into a body tissue, which can be moveably arranged on or at the infusion head. Furthermore the infusion head may comprise a coupling port for fluidic connection of the cannula to the infusion device. The cannula and the coupling port are arranged on a base element e. g. in form of pad, which can be attached to the tissue. The monitoring device can be arranged on the infusion head. The infusion head is part of a medical assembly comprising at least one further unit, which is moveable relative to the infusion head. The further unit may be designed as a preparation or storing unit interacting with the infusion head. The monitoring device is activated by connecting the infusion head to a coupler of the infusion device; by preparing or positioning the cannula in relation to the infusion head or the infusion device; or by opening a storage unit, which is housing the infusion head, or withdrawing the infusion head of the storage unit. These steps have to be executed during the general preparation of the infusion device for application. Therefore no special attention has to be paid to the activation of the monitoring device or to registration of data concerning the time period of application of the infusion head and the infusion device.

In one embodiment the second unit of a medical assembly comprising a disposable or replaceable infusion head can be realized by a coupler, which is releasably connectable to the coupling port of an infusion head. The coupler and the coupling port provide a fluidic connection between the infusion head and an infusion device. The coupling port and the coupler are provided with connection means and counter connection means designed for fluid tight engagement with each other. The engagement may be realized as snap connection, screw connection, a combination thereof or the like. The interaction area of the monitoring device may be provided on or adjacent to the coupling port or the coupler, e. g. the connection means or counter connection means. Of course such connection means and counter connection means may be realized for other medical assemblies than an infusion head or an infusion device within the scope of the invention.

In another embodiment the medical assembly may comprise a second unit realized by an operation housing, which at least partly covers the disposable or replaceable unit, in particular an infusion head, and interacts with a preparation mechanism of the disposable or replaceable unit, in particular a cannula mechanism, of the infusion head. Such a cannula mechanism is designed for moving the cannula relative to the infusion head from a storing position to an insertion position. In the storing position the cannula is protected by the operating housing or other casing of the infusion head and in an insertion position the cannula is exposed and ready for insertion. Therefore the cannula is moved relative to the base pad of the infusion head for example by a swivel or sliding mechanism as known from the art. Such cannula mechanism is operated by the operation housing, e. g. by pushing two housing portions into each other. One of the housing portions simultaneously presses on the interaction area of the monitoring device and starts counting and indicating the time period elapsed since the cannula has been exposed. Of course the operation housing could interact with other moveable parts of a disposable unit designed for preparation of the disposable unit for application in combination with a medical device.

Another example for use of the method according of the disclosure is a medical assembly comprising a blood glucose measuring device and test strip units, wherein the measuring strips are disposable material and have to be prepared for use with the measuring device. Also the medical assembly may comprise a case box for storing measuring strips for a blood glucose measuring device. The monitoring device can be arranged on the case box such that it is activated by opening and withdrawing a measuring strip.

### Brief description of the drawings

Some embodiments will be described in the accompanying drawings, which may explain the principles of the invention but shall not limit the scope of the invention. The drawings illustrate:

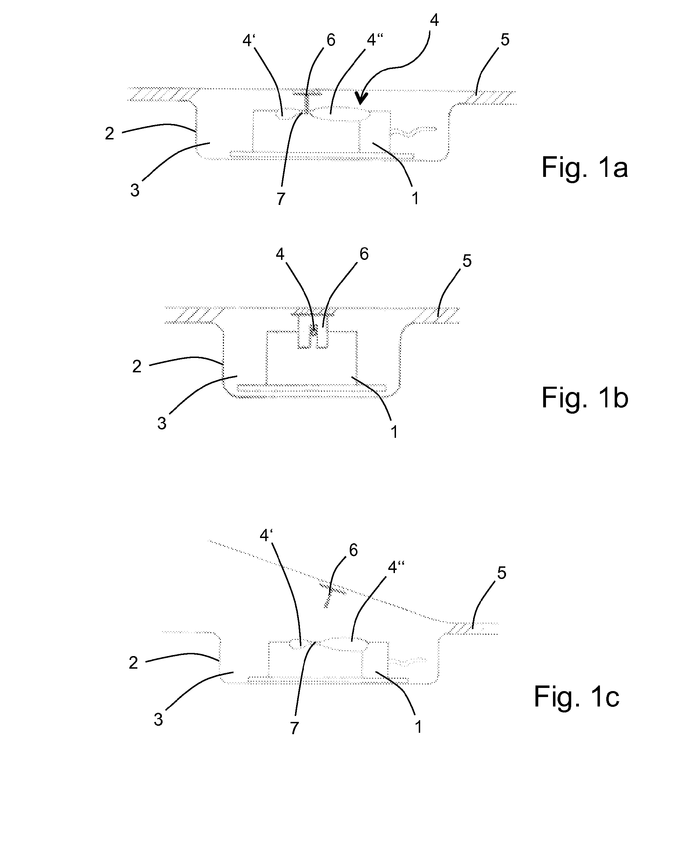

- Fig. 1 a: schematic view of a first embodiment of a medical assembly in a first state from a first side,
- Fig. 1 b: schematic view of the medical assembly according to figure 1 a in the first state from a second side,
- Fig. 1c: schematic view of the medical assembly according to figure 1a in a second state,
- Fig. 2a: schematic view of a second embodiment of a medical assembly in a first state,
- Fig. 2b: schematic view of the second embodiment of a medical assembly according to figure 2b in a second state,
- Fig. 3a: three-dimensional sectional view of a third embodiment of a medical assembly,
- Fig. 3b: three-dimensional sectional view of the third embodiment connected to a medical device,
- Fig. 4a: schematic view of a fourth embodiment of a medical assembly in a first state, and
- Fig. 4b: schematic view of the fourth embodiment in a second state.

In the following description of the drawings same elements in different embodiments and figures are referred to by the same reference numbers. Position indications like up, down, top bottom, left or right refer to positions or directions as given in the figures.

In figures 1a to 1c a first embodiment of a medical assembly is shown comprising a disposable unit in form of an infusion head 1. The infusion head 1 is stored in a second unit in form of a storing unit 2 comprising a storage compartment 3. The infusion head 1 rests on a base of the storage compartment 3. A monitoring device 4 is attached to the infusion head 1 on an upper side facing a closing foil 5 sealed to the storing unit 2 and closing the same. The monitoring device 4 comprises an activating chamber 4' storing a first reactant and an indication chamber 4" storing a second reactant. On the inner side of the closing foil 5 facing the infusion head 1 a separation means 6 is attached to the closing foil. The separation means 6 is sealed to the closing foil the same manner as the foil is sealed to the storing unit 2. The separation means 6 acts on the monitoring device at a connecting conduit 7 connecting the activating chamber 4' and the indication chamber 4" and blocks the connecting conduit 7 to separate the chambers 4' and 4".

In figure 1b the medical assembly is shown from another side as in figure 1a, which is turned about 90 degree. As shown the separation means 6 is designed as a fork means which picks up the monitoring device between the activating chamber 4' and the indication chamber 4". The separation means 6 separates the reactants within these chambers such that they cannot react or interact with each other. In this status the monitoring device is in an inactive state. The infusion head 1 is safely stored within the storing unit and may be stored for a long time in this state.

In figure 1c the storing unit has been opened by lifting the closing foil 5 for withdrawing the infusion head 1 from the storing unit 2, when the infusion head 1 is to be applied to a medical device, e. g. an infusion pump device. The storing unit, respectively the closing foil 5, has been moved relative to the disposable or replaceable infusion head 1 from the first position shown in figures 1a and 1b to a second position shown in figure 1c. By moving the closing foil, that means lifting the foil from the storing unit 2, the separation means 6 disengages off the monitoring device 4 of the infusion head 1 and frees the connecting conduit 7 between the activating chamber 4' and the indication chamber 4". The reactant of the activating chamber 4' may flow into the indication chamber 4" and successively react with the reactant in the indication chamber 4". That means the monitoring device 4 is in an active state. The reaction may cause a change of color, which is slowly moving in one direction within the indication chamber 4". The moving color change may indicate the period of time elapsed since the opening of the storing unit 2 and indicate the time remaining until the infusion head 1 has to be disposed. As soon as the storing unit 2 is opened to retract the infusion head 1 for application with an infusion device, the monitoring device 4 is automatically started. No additional activation step is necessary for activating the monitoring device.

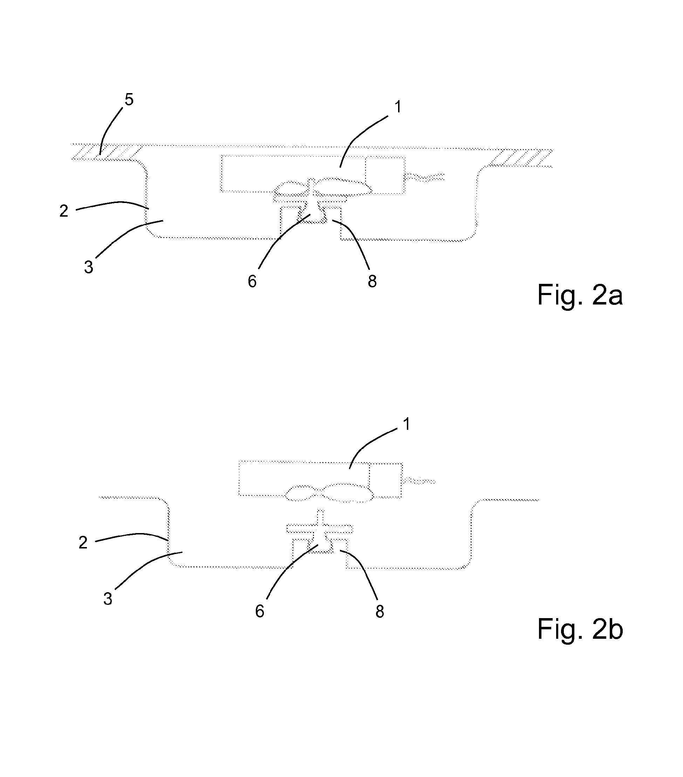

In figures 2a and 2b a second embodiment of a medical assembly is disclosed, which comprises a disposable or replaceable infusion head 1 and a storing unit 2, which are moveable relative to each other. The storing unit 2 is closed with a closing foil 5. On the base of the storage compartment 3 a separation means 6 is attached by a snap engagement with a socket 8 designed on the base for holding tightly the separation means 6. The separation means 6 again prevents the reactant of the activating chamber 4' to flow into the indication chamber 4" in this inactive state of the monitoring device 4 as explained in figures 1 a to 1 c.

In figure 2b the closing foil 5 has been removed from the storing unit 2 and the infusion head 1 is withdrawn from the storage compartment 3 to apply the infusion head 1 to an infusion device. By taking out the infusion head 1 of the storage compartment 3 the infusion head 1 is moved relative to the storing unit 2 and the monitoring device 4 is detached off the separation means 6, while the separation means 6 keeps engaged with the socket 8. Thus the reactants of the activating chamber 4' and indication chamber 4" are free to react with each other and the monitoring device 4 is in an activated state. That means as soon as the infusion head 1 is took out of the storing unit 1 to prepare the infusion device for application of infusion liquid, the monitoring device is activated automatically at the same time.

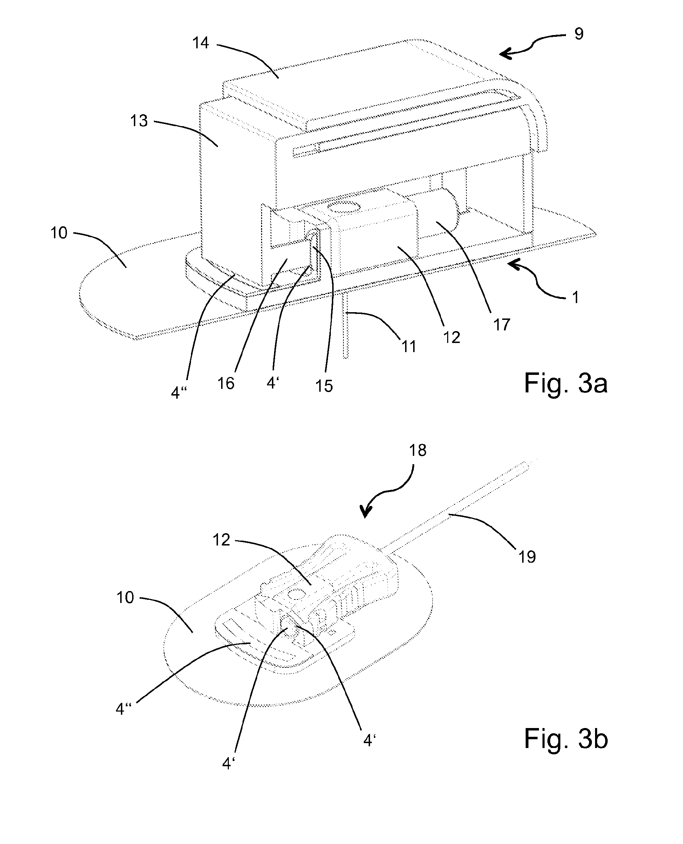

In figure 3a still another medical assembly is shown, which comprises a disposable or replaceable infusion head 1 and an operation housing 9, which is moveable relative to the infusion head 1. The infusion head 1 comprises a base pad 10, which is supposed to be attached to a body tissue of a user. Further the infusion head 1 is provided with a cannula 11, which is moveably arranged on a cannula casing 12. The casing comprises a coupling port 17, which is in fluidic connection with the cannula. The operation housing 9 sits on the base pad 10 and encloses the cannula casing 12 and the coupling port 17. The casing 12 houses a cannula mechanism, which is designed for moving the cannula 12 relative to the infusion head from a storing position to an insertion position. Therefore the operation housing 9 comprises a sliding portion 13 and a fixed portion 14. The fixed portion 14 is fixed relative to the base pad 10, while the sliding portion 13 is slideably arranged relative to the fixed portion 14 and to the infusion head 1 and may interact with the cannula mechanism. As soon as the sliding portion 13 is pushed into the fixed portion 14 the sliding portion 13 acts on a pusher, which actuates the cannula mechanism, which in turn moves the cannula from the storing position inside the casing 12 to an extended insertion position ready for insertion into a tissue a shown in figure 3a.

The pusher of the cannula mechanism comprises an interaction area 15, which is pressed by a ram 16 located on the sliding portion 13. The interaction area 15 is covered by an activating chamber 4' of the monitoring device. An indication chamber 4" is arranged on the base pad 10 and is separated to the activating chamber 4' in a first state, in which the pusher of the cannula mechanism is not under pressure of the ram of the sliding portion 13. As soon as the sliding portion 13 presses against the interaction area 15 and the activating chamber 4' as shown in figure 3a, the generated pressure in the activating chamber 4' results in deblocking a conduit to the indication chamber and pressing the reactant in direction of the indication chamber 4" through a connection layer. Thus the two reactants may react in the indication chamber 4" and the monitoring device is in an activated state.

In a first position of the sliding portion 13, in which the sliding portion 13 extends out of the fixed portion 14, the sliding portion 13 covers the indication chamber 4" on the base pad 10. The indication chamber 4" may also be covered in a second position, in which the sliding 13 portion is pushed into the fixed portion 14 and the pusher is pressed. The operation housing 9 can be detached of the base pad 10 and disposed. That means the operation housing 9 can also be regarded as a disposable unit in the sense of the present disclosure.

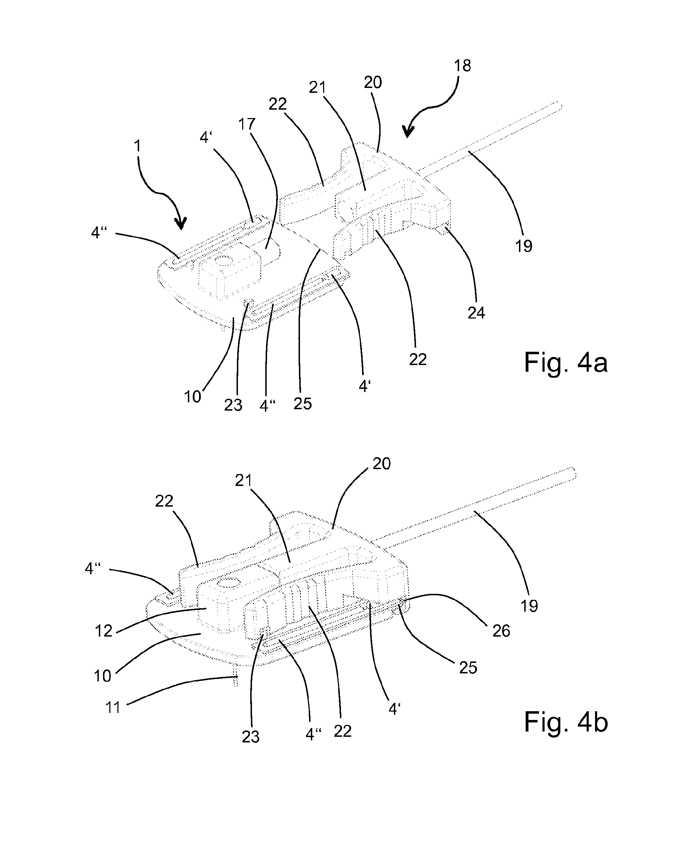

As soon as the operation housing 9 is removed of the infusion head 1 the casing 12 and the coupling port 17 are accessible and also the indication chamber 4" is fully visible as can be seen in figure 3b. The coupling port 17 is connected to a coupler 18 comprising an infusion tube 19. The coupling may for example be realized by an attachment mechanism, which will be explained in more detail in figures 4a and 4b. Thus the infusion head 1 is connected to a medical device (not shown); the infusion device in this embodiment. The infusion tube 19 is in fluidic connection with the cannula via the coupling port 17 and casing 12. The indication chamber 4" extends in form of a longitudinal strip on one side of the base pad 10 nearly over the full length of the base pad. The reactant of the activating chamber 4' travels to the reactant of indication chamber 4" resulting in reaction of the two reactants such that a color indication is realized, which travels along the length of the indication chamber 4". The indication chamber 4" indicates the time period remaining until disposal of the infusion head 1 by a color indication.

In figures 4a and 4b another embodiment of the medical assembly is illustrated comprising a disposable or replaceable infusion head 1 and a coupler unit in form of the coupler 18, which is moveable relative to the infusion head 1 to connect an infusion device to the infusion head 1 and prepare the infusion device for application. Therefore the coupler 18 comprises a main body 20 with a connection port 21 and a resilient attachment arm 22 on each side of the port 21 extending parallel along the axis of the infusion tube 19 towards the infusion head 1. The attachment arms 22 are designed to snap between attachment pins 23 extending upward from the base pad 10. The attachment arms 22 snap into a fixation position with the pins 23 and engage behind the pins 23 as can be seen in figure 4b. The main body 20 comprises a groove 24 running parallel to a positioning edge 25 of the base pad 10. A monitoring device 4 comprises two activating chamber 4' and two indication chamber 4". The two activating chambers 4' are located close to the positioning edge 25 while the indication chambers 4" reach over the length of the base pad 10 on two opposing sides of the base pad 10 perpendicular to the positioning edge 25.

In figure 4a the infusion head 1 and the coupler 18 take a first position relative to each other, wherein the coupling port 17 and the connection port 21 are separated. The monitoring device 4 is in an inactive state. In figure 4b the coupler 18 is attached to the infusion head 1. Therefore the coupler 18 is slid upon the base pad 10 of the infusion head 1 such that the attachment arms 22 engage with the attachment pins 23 and the positioning edge 25 is slid into the groove 24 such that the surface of the main body 20 presses on the activating chambers 4' on both sides of the pad 10. Therefore the main body 10 may comprise an inclined plane 26 along which the positioning edge 25 is guided into the groove 24, and which squeezes the activating chambers 4" in the attachment position. That means the infusion head 1 and the coupler 18 take a second position, the monitoring device 4 is in an active state and the medical assembly is connected to an infusion device and ready for use. The generated pressure in the activating chambers 4" cause reactant in the activating chambers 4" to pass to the indication chambers 4" and react with the reactant in the indication chambers. Thus an indication line moves along the length of both indication chambers 4" and indicates a period of time for example for replacing the infusion head 1 on the coupler 18. The two indication chambers 4" on both sides of the casing 12 and the coupler 18 enables a user to view the monitoring device irrespective of the orientation of the infusion head 1 on a body tissue. The monitoring device is automatically activated, as soon as the coupler 18 is attached to the infusion head 1 to prepare the infusion device for application.

The medical assembly and the method according to the invention have been illustrated by means of advantageous embodiments, which shall not limit the scope of invention. It will be apparent to a person skilled in the art of medical devices, that other embodiments of medical assembly may advantageously use the method according of the invention.

### LIST OF REFERENCE NUMBERS

- 1: infusion head
- 2: storing unit
- 3: storage compartment
- 4: monitoring device
- 4': activating chamber
- 4": indication chamber
- 5: closing foil
- 6: separation means
- 7: connecting conduit
- 8: socket
- 9: operation housing
- 10: base pad
- 11: cannula
- 12: casing
- 13: sliding portion
- 14: fixed portion
- 15: interaction area
- 16: ram
- 17: coupling port
- 18: coupler
- 19: infusion tube
- 20: main body
- 21: connection port
- 22: attachment arm
- 23: attachment pins
- 24: groove
- 25: positioning edge
- 26: inclined plane

## Claims

1. Disposable medical device (1) for use in combination with a further medical device comprising a monitoring device (4) for counting and/or displaying a time period of application and/or remaining before required disposal or replacement of the disposable or replaceable unit, the monitoring device (4) being designed to switch, upon activation, from an inactive state to an active state where its counts and/or displays the time period of application and/or remaining before required disposal, wherein the disposable medical device (1) is designed such that the monitoring device (4) is automatically activated along with carrying out a preparatory or handling step that is required for preparing the disposable medical device (1) for use.

2. Disposable medical device according claim 1, **characterized in that** the monitoring device (4) is pressure sensitive.

3. Disposable medical device according to claim 1 or 2, **characterized in that** the monitoring device (4) comprises an interaction area (15), which is automatically activated along with carrying out the preparatory or handling step.

4. Disposable medical device according to one of claims 1 to 3, **characterized in that** the monitoring device (4) comprises at least one activating chamber (4') including a first reactant and at least one indication chamber (4") including a second reactant, wherein the activating chamber (4') and the indication chamber (4") are separated before carrying out the preparatory or handling step and are connected after carrying out the preparatory or handling step.

5. Disposable medical device according to claim 4, **characterized in that** after carrying out the preparatory or handling step first and second reactants react with each other such that a visible indication moves along an axis of the indication chamber (4") in a predetermined period of time.

6. Disposable medical device according to one of the preceding claims, **characterized in that** the monitoring device (4) is sealed into a surface (10) of the disposable medical device (1).

7. Disposable medical device according to one of the preceding claims, **characterized in that** the disposable medical device (1) is designed as an infusion head (1) to be placed on a tissue of a patient for administration of medical or pharmaceutical liquid into the tissue comprising a connected or connectable cannula (11).

8. Disposable medical device according to claim 7, **characterized in that** an indication chamber (4") is provided on at least two opposing sides of the infusion head (1).

9. Disposable medical device according to one of the preceding claims, **characterized in that** the device comprises a housing (9), which at least partly covers the infusion head (1), is moveable relative to the cannula (11) and interacts with the cannula (11) of the infusion head (1).

10. Medical assembly comprising at least two units (1; 2; 9; 18), which are moveable relative to each other, wherein at least one of the units is a disposable unit or device (1) designed for application in combination with a medical device, and a chemical or physical monitoring device (4) for counting and/or displaying a time period of application and/or remaining before required disposal or replacement of the disposable unit ,
**characterized in that** in an inactive state the at least two units (1; 2; 9; 18) take a first position relative to each other and the monitoring device (4) is inactive and in an active state the at least two units (1; 2; 9; 18) take a second position relative to each other and the monitoring device (4) is active, wherein the monitoring device (4) is automatically activated to count and/or display the time period by movement of the at least two units (1; 2; 9; 18) relative to each other from the first to the second position.

11. Medical assembly according to claim 15, **characterized in that** the monitoring device (4) is arranged on a disposable unit in form of a disposable medical device (1) according to one of claims 1 to 9.

12. Medical assembly according to one of claims 10 or 11, **characterized in that** first or second unit acts on the interaction area and activates the monitoring device (4), when moved from first to second position.

13. Medical assembly according to one of claims 10 to 12, **characterized in that** the second unit is realized by a storage unit (2) for storing the first unit (1), which comprises a separator means (6) for separating the activating chamber (4') and the indication chamber (4").

14. Medical assembly according to claim 13, **characterized in that** the separator means (6) is provided on a base of the storing unit (2), wherein the monitoring device (4) of the disposable unit (1) rests on the separator means (6) in the first state.

15. Medical assembly according to claim 13, **characterized in that** the separator means (6) is provided on a closing foil (5) of the storing unit (2) and engages with the monitoring device (4) in a closed state of the storing unit (2).

16. Medical assembly according to one of claims 10 to 15, **characterized in that** the disposable unit (1) is designed as an infusion head (1) and the second unit is designed as a preparation or storing unit (2; 9; 18) interacting with the infusion head (1).

17. Medical assembly according to one of claims 10 to 16, **characterized in that** the second unit is realized by a coupler (18), which is releasably connectable to a coupling port (17) of the infusion head (1).

18. Method of preparing at least parts of a medical device for application, wherein the medical device comprises at least one disposable unit or device (1) and a chemical or physical monitoring device (4) for counting and/or displaying a time period of application and/or remaining before required disposal or replacement of the disposable unit (1), **characterized by** automatically activating the monitoring device (4) by moving the at least one disposable unit (1) relative to a second unit (2; 9; 18) for:
- preparing or positioning the disposable unit (1) for use with the medical device;
- assembling the at least one disposable unit (1) and the medical device; and/or
- withdrawing the disposable unit (1) of a storage compartment (3) of the second unit.

19. Method according to claim 18, wherein the medical device is realized by an infusion device comprising a disposable unit in form of an infusion head (1), wherein the monitoring device (4) is activated by:
- connecting the infusion head (1) to a coupler (18) of the infusion device;
- preparing or positioning a cannula (11) moveably arranged on/at the infusion head (1); or
- opening a storage unit (2), which is housing the infusion head (1), or withdrawing the infusion head (1) of the storage unit (2).
